# EUROPEAN PATENT SPECIFICATION

(11) **EP 0 294 392 B1**
(45) Date of publication and mention of the grant of the patent: **25.10.1995**
(21) Application number: 87901952.9
(22) Date of filing: 25.02.1987
(51) Int. Cl.: C07K 2/00, C07K 17/06, C07K 17/10, A61K 31/705, A61K 39/44

(54) **DIAGNOSTIC AND THERAPEUTIC ANTIBODY CONJUGATES**
DIAGNOSE- UND HEILMITTEL-ANTIKÖRPERKONJUGATE
CONJUGUES DIAGNOSTIQUES ET THERAPEUTIQUES ASSOCIES A UN ANTICORPS

(30) Priority: 25.02.1986 US 833204
(43) Date of publication of application: 14.12.1988
(73) Proprietor: CENTER FOR MOLECULAR MEDICINE AND IMMUNOLOGY, Newark, NJ 07103 (US)
(72) Inventor: SHIH, Lisa, B., Cedar Grove, NJ 07009 (US); PRIMUS, F., James, Pittstown, NJ 08867 (US); GOLDENBERG, M., David, Short Hills, NJ 07078 (US)
(74) Representative: Sheard, Andrew Gregory
(86) International application number: PCT/US87/00406
(87) International publication number: WO 87/05031

(56) References cited:
- EP-A- 0 088 695
- EP-A- 0 112 720
- US-A- 415 665
- US-A- 4 046 722
- US-A- 4 093 607
- US-A- 4 263 279
- US-A- 4 315 851
- INTERNATL. JOURNAL OF CANCER, vol. 41, 1988; pp. 832-839
- INTERNATL. JOURNAL OF CANCER, vol. 46, 1990; pp. 1101-1106
- CANCER RESEARCH, vol. 51, 1991; pp. 4192-4198
- CANCER IMMUNOTHERAPY, vol. 31, 1990; pp. 197-201

## Description

### BACKGROUND OF THE INVENTION

The present invention relates to conjugates of diagnostic or therapeutic principles, such as drugs, toxins, chelators, boron compounds and detectable labels, to an antibody, in which the diagnostic or therapeutic principle is first loaded onto a polymer carrier such as an aminodextran or a polypeptide of at least 50̸ amino acids in length, and this intermediate is in turn site-specifically conjugated to a targeting antibody such as an antitumor antibody. The resultant conjugate targets the diagnostic or therapeutic principle to a target tissue or organ where the diagnostic or therapeutic effect is realized.

Conjugation of cytotoxic drugs to antibodies to achieve a targeted therapeutic result is known. In particular, it is known that methotrexate (MTX) can be conjugated to antibodies and some selective cytotoxicity has been observed. It is desirable to enhance the selectivity and cytotoxicity of such conjugates by increasing the antibody loading of the cytotoxic drug. However, multiple conjugation of individual drug molecules to an antibody eventually reduces its immunoreactivity, the effect being observed when more than about ten drug molecules are loaded.

It has been suggested that the drug be conjugated to an intermediate polymeric carrier, which in turn would be conjugated to antibody. This has the advantage that larger numbers of drug molecules can be attached to the antibody at fewer sites on the antibody itself, so that immunoreactivity is not as seriously compromised.

One approach has been to attach MTX to bovine serum albumin (BSA) and then randomly link the intermediate to antibody, as reported by Garnett et al., Int. J. Cancer, 31:661-670̸, 1983. These authors were able to attach about 37 MTX molecules to BSA (average molecular weight of 70̸,0̸0̸0̸) but the resultant antibody conjugate had an immunoreactivity of only about 28% of that of the intact antibody.

Use of polylysine as a polymer carrier was reported by Ryser et al., Proc. Natl. Acad. Sci. USA, 75:3867-3870̸, 1978. These authors found that only about 13 MTX per carrier could be loaded and immunoreactivity was poor. In addition, the high amine content of the polymer, largely in the form of charged ammonium groups, caused the conjugate to stick to normal cells and vitiated the selectivity of the cytotoxic effect.

Rowland, U.S. Patent 4,0̸46,722, discloses an antibody conjugate wherein a plurality of molecules of a cytotoxic agent are covalently bound to a polymer carrier of molecular weight 50̸0̸0̸-50̸0̸,0̸0̸0̸, and the loaded carrier is covalently bound to an antibody by a random attachment to pendant amine or carboxyl groups. The covalent attachment is effected either by direct condensation to form amide bonds between amine groups on one and carboxyl groups on the other component of the conjugate, or by glutaraldehyde linkage of amine groups on the carrier to amine groups on the antibody. Again, this has the disadvantages of loss of immunoreactivity of the antibody and some risk of crosslinking of the antibody and/or the carrier. Ghose et al., J Natl Cancer Inst, 61:657-676, 1978, disclose other antibody-linked cytotoxic agents useful for cancer therapy, but again, covalent attachment is not to the oxidized carbohydrate portion of the antibody. These references show that it is well known in the art to prepare drug-loaded polymer carriers, but that their mode of attachment to antibodies in the past has been random, through pendant amine or carboxyl groups on the antibody.

Chelating groups for radiometals and/or metal ions which can act as magnetic resonance enhancing agents have been covalently bound to antibodies by a variety of methods, most involving random attachment to pendant amine, carboxyl, sulfhydryl, or phenyl groups on the polypeptide chain. Toxins and boron addends have also been linked to antibodies by various methods for targeted therapy, the boron groups being activated by thermal neutron irradiation once they have been localized at the site of a tumor or other pathological lesion by the antibody targeting vehicle to which they are bound. Detectable labels, such as enzymes, DNA segments, fluorescent compounds and the like, have been bound to antibodies for use in assays, again by random coupling to pendant groups.

In an attempt to avoid the undesirable effects of random coupling to antibody and crosslinking, McKearn, et al., in European Patent Application No. 88,695, published on Sept. 14, 1983, disclose a method for preparing antibody conjugates which involves oxidizing the carbohydrate portion of the antibody and linking compounds with free amine groups to the resultant carbonyls (aldehyde and/or ketone groups) by Schiff base formation and optional reductive stabilization. This reference discloses site-specific attachment of a variety of compounds, such as chelators, drugs, toxins, detectable labels and the like, to the oxidized carbohydrate portion of an antibody. Short peptide linkers are disclosed to provide spacers between these compounds and the antibody, either to make the linkage more readily cleaved or resistant to cleavage at the target site. Attachment of the oxidized carbohydrate to a polymer such as an aminodextran is disclosed, but only in the context of linking the antibody to an insoluble support such as a polymer coated bead, plate or tube, such as would be used in an immunoassay. There is no suggestion in this reference to covalently bind a polymer carrier loaded with functional molecules such as drugs, chelators or the like, to the oxidized carbohydrate portion of an antibody to prepare a soluble conjugate for use as a diagnostic or therapeutic agent.

A need therefore continues to exist for an antibody conjugate of a diagnostic or therapeutic principle, such as a drug, toxin, chelator, boron compound or detectable label, that combines high loading with minimal decrease in immunoreactivity for selective targeting of the diagnostic or therapeutic principle to a target tissue or organ, or for highly efficient and sensitive immunoassay or immunohistological applications.

### OBJECTS OF THE INVENTION

One object of the present invention is to provide a conjugate of a diagnostic or therapeutic principle to an targeting antibody wherein a plurality of molecules of the diagnostic or therapeutic principle are attached to the antibody, for enhanced diagnostic or therapeutic effect at the target site.

Another object of the invention is to provide a conjugate of a plurality of molecules of a diagnostic or therapeutic principle to an antibody wherein the conjugate has substantially the same immunoreactivity as intact antibody.

Another object of the present invention is to provide a conjugate of an antitumor or antibiotic drug or toxin to an anticancer or antilesion antibody, wherein the conjugate does not appreciably bind to non-target cells or tissues, and wherein the targeted conjugate can enter the tumor cell or site of infection or release its therapeutic principle at the target site, and achieve its tumoricidal or antibiotic effect at the target, while minimizing the systemic side effects of the drug or toxin.

Another object of the invention is to provide an antibody conjugate comprising a plurality of chelators, for use as a targeted imaging agent for scintigraphic or magnetic resonance imaging, or for use as a targeted therapy agent for radioisotope therapy.

Another object of the invention is to provide an antibody conjugate comprising a plurality of boron addends, for use as a neutron activation therapy agent.

Another object of the invention is to provide an antibody conjugate comprising a plurality of detectable labels, for use as a reagent for immunoassay or immunohistology.

Another object of the invention is to provide a method of producing antibody conjugates having the aforementioned properties.

Another object of the present invention is to provide methods of diagnosis and therapy using the foregoing antibody conjugates for targeted delivery of diagnostic and therapeutic principles to sites of lesions and infections, or using the conjugates to carry a plurality of detectable labels for enhanced detection in immunoassay or immunohistological applications.

Upon further study of the specification and appended claims, further objects and advantages of this invention will become apparent to those skilled in the art.

### SUMMARY OF THE INVENTION

These objects can be attained by providing an antibody conjugate, comprising a plurality of drug, toxin, chelator, boron addend or detectable label molecules covalently bound to an aminodextran carrier having at least one remaining free amine group, the loaded carrier in turn being covalently bound through said at least one amino group to the carbohydrate portion of an antibody by a reduced Schiff base linkage.

The invention further includes a method of preparing an antibody conjugate, comprising the steps of:
(a) reacting an aminodextran carrier to which are covalently bound a plurality of drug, toxin, chelator, boron addend or detectable label molecules, said carrier having at least one remaining free amine group, to an antibody having an oxidized carbohydrate portion; and
(b) reductively stabilizing the resultant Schiff base adduct.

Also included in the invention are improved methods of diagnosis and therapy using the antibody conjugates of the invention.

### DETAILED DISCUSSION

The general method of preparing an antibody conjugate according to the invention involves reacting an antibody, whose carbohydrate portion has been oxidized, with a carrier aminodextran loaded with a plurality of drug, toxin, chelator, boron addend or detectable label molecules, and having at least one remaining free amine function. This results in an initial Schiff base (imine) linkage, which is stabilized by reduction to a secondary amine to form the final conjugate.

The carrier polymer is an aminodextran (AD). It is desirable for the final antibody conjugate to be soluble in human serum, for ease of administration and efficient targeting, when used as an in vivo diagnostic or therpeutic agent. Thus, solubilizing functions on the carrier polymer will enhance the serum solubility of the final conjugate. In particular, an aminodextran with hydroxyl functions on the amine portion will be preferred.

The process for preparing a conjugate with an aminodextran carrier normally starts with a dextran polymer, advantageous, a dextran of average molecular weight (MW) of about 10̸,0̸0̸0̸ - 10̸0̸,0̸0̸0̸, preferably about 30̸,0̸0̸0̸ - 60̸,0̸0̸0̸, and more preferably about 40̸,0̸0̸0̸. The dextran is then reacted with an oxidizing agent to effect a controlled oxidation of a portion of its carbohydrate rings to generate aldehyde groups. The oxidation is conveniently effected with glycolytic chemical reagents, e.g., NaIO₄, according to conventional procedures.

It is convenient to adjust the amount of oxidizing agent so that about 50̸ - 150̸, preferably about 10̸0̸ aldehyde groups are generated, for a dextran of MW of about 40̸,0̸0̸0̸, with about the same proportion of aldehyde groups for other MW dextrans. A larger number of aldehyde groups, and subsequent amine groups, is less advantageous because the polymer then behaves more like polylysine. A lower number results in less than desirable loading of drug, toxin, chelator, boron addend or label molecules, which may be disadvantageous.

The oxidized dextran is then reacted with a polyamine, preferably a diamine, and more preferably a mono- or polyhydroxy diamine. Suitable such amines include, e.g., ethylene diamine, propylene diamine or other like polymethylene diamine, diethylene triamine or like polyamines, 1,3-diamino-2-hydroxypropane or other like hydroxylated diamine or polyamine, and the like. Earlier workers have generally used ethylene diamine, but the present inventors have shown that better results are achieved with a solubilizing diamine such as 1,3-diamino-2-hydroxypropane. An excess of the amine relative to the aldehyde groups is used, to insure substantially complete conversion of the aldehyde functions to Schiff base (imine) groups.

Reductive stabilization of the resultant intermediate is effected by reacting the Schiff base intermediate with a reducing agent. e.g., NaBH₄, NaBH₃CN or the like. An excess of the reducing agent is used to assure substantially complete reduction of the imine groups to secondary amine groups, and reduction of any unreacted aldehyde groups to hydroxyl groups. The resultant adduct can be further purified by pasage through a convential sizing column to remove cross-linked dextrans. An estimate of the number of available primary amino groups on the AD can be effected by reaction of a weighed sample with trinitrobenzenesulfonic acid and correlation of the optical density at 420̸ nm with a standard. This method normally results in essentially complete conversion of the calculated number of aldehyde groups to primary amine groups on the AD.

Other conventional methods of derivatizing a dextran to introduce amine functions can also be used, e.g., reaction with cyanogen bromide, followed by reaction with a diamine.

The AD is then reacted with a derivative of the particular drug, toxin, chelator, boron addend or label to be loaded, in an activated form, preferably a carboxyl-activated derivative, prepared by conventional means, e.g., using dicyclohexylcarbodiimide (DCC) or a water soluble varient thereof, to form an intermediate adduct.

Methotrexate (MTX) is a typical drug for use in preparing conjugates according to the invention and will be used to illustrate the procedure. Analogous steps will be used for other drugs, toxins, chelators, boron addends and labels, modified in appropriate ways which will be readily apparent to the ordinary skilled artisan. Activation of MTX is conveniently effected with any of the conventional carboxyl-activating reagents, e.g., DCC, optionally followed by reaction with N-hydroxysuccinimide (HOSu), to form the active ester. The reaction is normally effected in a polar, aprotic solvent, e.g., dimethylformamide (DMF), dimethylsulfoxide (DMSO) or the like. Other activated esters, e.g., p-nitrobenzoate and the like, can also be used, as can mixed anhydrides. The DCC/HOSu activation is mild and the activated MTX can be reacted in aqueous medium with the AD, so it is preferred.

The proportions of activated MTX to AD are preferably such that about half of the amino groups available on the AD react to form amide bonds with the carboxyl of the activated MTX. Thus, if about 10̸0̸ amine groups are available on an AD with a starting MW of about 40̸,0̸0̸0̸, up to about 50̸ of these should be reacted with activated MTX. Using a proportion of about 50̸:1 MTX:AD, about 25-50̸ MTX molecules arc normally introduced. It is difficult to achieve higher loading because of incipient precipitation of the adduct due to the increasing insolubility thereof.

As an illustration of the adaptations to be used for other drugs, loading with 5-Fluorouracil (5-FU) can be effected by oxidizing 5-Fluorouridine at the carbohydrate, e.g., using periodate, reacting this intermediate with an aminodextran, and reductively stabilizing the Schiff base adduct. Cycloheximide can be loaded by direct reaction of its cyclohexanone carbonyl with aminodextran amine groups, followed by reductive stabilization, or by reacting its side chain hydroxyl with an excess of a diisothiocyanate linker and reaction of the isothiocyanate derivative with amines on the aminodextran, or by reaction of the imide nitrogen with, e.g., a haloacid or haloester, followed by activation of the resultant carboxyl derivative, e.g. with DCC, and condensation with amines on the aminodextran.

Another illustration is provided by the antibiotic mitomycin C and its analogues. This molecule has an amine function and a cyclic imine, either of which can be reacted with an alkylating activating group, e.g., succinimidyloxy iodoacetate or sulfosuccinimidyloxy (4-iodoacetyl)aminobenzoate (sulfo-SIAB), the resulting intermediate is then used to alkylate amine groups on an aminodextran. Alternatively, carboxyl groups can be introduced using, e.g., succinic anhydride, then activated, e.g., with DCC, and the activated intermediate coupled as before.

Toxins, e.g., pokeweed antiviral protein (PAP) or the ricin A-chain, and the like, can be coupled to aminodextrans by glutaraldehyde condensation or by reaction of activated carboxyl groups on the protein with amines on the aminodextran.

Many drugs and toxins are known which have a cytotoxic effect on tumor cells or microorganisms that may infect a human and cause a lesion, in addition to the specific illustrations given above. They are to be found in compendia of drugs and toxins, such as the Merck Index and the like. Any such drug can be loaded onto the AD or PP by conventional means well known in the art, and illustrated by analogy to those described above.

Chelators for radiometals or magnetic resonance enhancers are also well known in the art. Typical are derivatives of ethylenediaminetetraacetic acid (EDTA) and diethylenetriaminepentaacetic acid (DTPA). These typically have groups on the side chain by which the chelator can be attached to a carrier. Such groups include, e.g., a benzylisothiocyanate, by which the DTPA or EDTA can be coupled to the amine group of an antibody. In the present invention, this same group would be used to couple the chelator to amine groups on an AD or PP. Alternatively, carboxyl groups or amine groups on a chelator can be coupled to an AD or a PP by activation or prior derivatization and then coupling, all by well known means. For example, deferoxamine, which is a chelator for Ga-67, has a free amine group that can be coupled to an activated carboxyl of a PP containing glutarate or aspartate residues, or can be activated with a suitable linker to contain an activated carboxyl, isothiocyanate or like group, and then coupled to amines on an AD or a PP with lysine residues therein.

Other methods of linking chelators to amines of an AD will be apparent to the skilled artisan, depending upon the functionality of the chelator. Such linkages to amine or carboxyl groups of antibodies is known, and can be adapted readily to linkage to AD or PP carrier polymers. Linkage to other functions on the chelator will also be apparent. e.g., to sulfhydryls by alkylation to form thioethers, to hydroxyls by acylation to preferably form urethanes or to form esters, to aromatic rings by diazonium coupling to a group which can then be further converted to a carboxyl or amine for coupling to carrier.

Labels such as enzymes, fluorescent compounds, electron transfer agents, and the like can also be linked to carrier by conventional methods well known to the art. These labeled carriers and the antibody conjugates prepared from them can be used for immunoassays and for immunohistology, much as the antibody conjugate prepared by direct attachment of the labels to antibody. However, the loading of the conjugates according to the present invention with a plurality of labels can increase the sensitivity of assays or histological procedures, where only a low extent of binding of the antibody to target antigen is achieved.

Boron addends, e.g., carboranes, when attached to antibodies and targeted to lesions, can be activated by thermal neutron irradiation and converted to radioactive atoms which decay by alpha emission to produce highly cytotoxic short-range effects. High loading of boron addends, as well as of magnetic resonance enhancing ions, is of great importance in potentiating their effects. Carboranes can be made with carboxyl functions on pendant side chains, as is well known in the art. Attachment of these carboranes to AD's or PP's by activation of the carboxyl groups and condensation with amines on the carriers enables preparation of useful loaded carriers.

Conjugation of the adduct with the antibody is effected by oxidizing the carbohydrate portion of the antibody and reacting the resultant aldehyde (and ketone) carbonyls with amine groups remaining on the carrier after loading, or introduced thereon after loading, with the drugs, toxins, chelators, boron addends or labels. Typically, for an AD, not all of the amines of the AD are use to load the carrier, and remaining amines condense with the oxidized antibody to form Schiff base adducts, which are then reductively stabilized, normally with a borohydride reducing agent.

For example, the MTX-AD adduct can be conjugated to any antibody which specifically binds to an antigen produced by or associated with a tumor which is responsive to methotrexate therapy. Examples of such antigens are human chorionic gonadotropin (HCG), carcinoembryonic antigen (CEA), alpha-fetoprotein (AFP), breast gross cystic disease protein, breast epithelial cell antigen, and other breast, lung, ovarian, germ cell, brain, lymphoma and leukemia antigens. Antibodies to such antigens can be developed by immunizing a suitable animal host with purified anti-cancer antigens or tumor or normal organ/tissue extracts and/or cells.

These antigens and/or cells/extracts can also be used in conventional methods of producing hybridomas which produce monoclonal antibodies. Human or primate hybridoma monoclonal antibodies can be produced by a combination of genetic engineering and hybridoma technology.

The next step involves oxidation of the carbohydrate portion of the antibody chosen for the conjugate. This is conveniently effected either chemically, e.g., with NaIO₄ or other glycolytic reagent, or enzymatically, e.g., with neuraminidase and galactose oxidase. The latter is a convenient method which is well known for linking amino moieties to antibodies, e.g., as reported by Banjo et al., Int. J. Cancer, 13:151-163, 1974.

The proportion of oxidized antibody and MTX-AD adduct are adjusted so that an average of about 1 - 3 adducts are linked to the antibody. This will result in a MW for the conjugate of less than about 30̸0̸,0̸0̸0̸, which is desirable to promote adequate loading without interfering with cellular uptake and diffusion into solid tumor, and at the same time avoiding or at least mitigating rapid clearance of the conjugate from the blood stream. Attachment of the MTX-AD conjugate to the antibody in a site-specific manner on the carbohydrate portion of the molecule preserves the antibody binding activity, while at the same time permitting a high loading of the drug.

Loading of drugs on the carrier will depend upon the potency of the drug, the afficiency of antibody targeting and the efficacy of the conjugate once it reaches its target. In most cases, it is desirable to load at least 20̸, preferably 50̸ and often 10̸0̸ or more molecules of a drug on a carrier. The ability to partially or completely detoxify a drug as a conjugate according to the invention, while it is in circulation, can reduce systemic side effects of the drug and permit its use when systemic administration of the unconjugated drug would be unacceptable. For example, MTX and cycloheximide often are too toxic when administered systemically. Administration of more molecules of the drug, but conjugated to antibody on a carrier, according to the present invention, permits therapy while mitigating systemic toxicity.

Toxins will often be less heavily loaded than drugs, but it will still be advantageous to load at least 5, preferably 10̸ and in some cases 20̸ or more molecules of toxin on a carrier and load at least one carrier chain on the antibody for targeted delivery.

As noted above, it is often highly advantageous to load many molecules of a chelator on a carrier to form a conjugate especially when the metal ion to be chelated is a paramagnetic ion for magnetic resonance enhancement. In such cases, higher molecular weight carrier polymer chains are preferably used and more than one loaded carrier is condensed to the carbohydrate portion of the antibody. For example, using an AD made from a dextran of average molecular weight of 10̸0̸,0̸0̸0̸, preferably an AD prepared with 2-hydroxy-1,3-diaminopropane, and preferably containing about 10̸0̸-20̸0̸ amine groups per dextran about 10̸0̸ DTPA chelator groups are coupled, using either a conventional cyclic-DTPA procedure or by reaction with excess of another derivative with an activated carboxyl on a side chain or with another reactive acylating group on the side chain, e.g., an isothiocyanate, or with an alkylating function on the side chain, e.g., an alpha-iodobenzyl or an iodoalkyl group. Coupling of several loaded carrier polymer chains, preferably 1.5-4 chains, to the oxidized carbohydrate portion of antibody will permit loading of 150̸-40̸0̸ paramagnetic ions on a single antibody after the chelator conjugate is loaded with the metal ions, by conventional means, preferably in metal-free solution and using a transchelation agent.

Administration of antibody conjugates of the invention for in vivo diagnostic and therapeutic applications will be by analogous methods to conjugates of the same or similar drugs, toxins, chelators or boron adducts where the diagnostic or therapeutic principle is directly linked to antibody or a loade carrier is linked by random binding to amine or carboxyl groups on amino acid residues of the antibody in a non-site-specific manner. Such modes of administration are illustrated in references already cited herein for illustrative purposes, and are also found widely in the literature, so that they will be well known to the skilled artisan. More precise dosimetry will be necessary for each agent, again as is well known in the art.

Administration of the MTX-AD-Ab can be effected in a variety of ways depending upon the type and location of the tumor to be treated. For example, administration can be intravenous, intraarterial, intraperitoneal, intrapleural, intrathecal, subcutaneous, by perfusion through a regional catheter, or by direct intralesional injection.

The conjugate will generally be administered as a sterile aqueous solution in phosphate-buffered saline. Dosage units of about 10̸-20̸0̸ mg of conjugate will be administered, normally daily for a period of several days. It may be necessary to reduce the dosage and/or use antibodies from other species and/or hypoallergenic antibodies, e.g., hybrid human or primate antibodies, to reduce patient sensitivity.

Intravenous, intraarterial or intrapleural administration is normally used for lung, breast, and leukemic tumors. Intraperitoneal administration is advised for ovarian tumors. Intrathecal administration is advised for brain tumors and leukemia. Subcutaneous administration is advised for Hodgkin's disease, lymphoma and breast carcinoma. Catheter perfusion is useful for metastatic lung, breast or germ cell carcinomas of the liver. Intralesional administration is useful for lung and breast lesions.

The above illustration will show the general methods of administration of conjugates according to the present invention. Conjugates of boron addend-loaded carrier for thermal neutron activation therapy will normally be effected in similar ways, and it will be advantageous to wait until non-targeted conjugate clears before neutron irradiation is performed. Such clearance can be accelerated by use of second antibody, as is known from, e.g., U.S. Patent 4,624,846.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever. In the following examples, all temperatures are set forth uncorrected in degrees Celsius; unless otherwise indicated, all parts and percentages are by weight.

### Example 1

### Preparation of amino dextran

One gram of dextran (MW 40̸,0̸0̸0̸, Sigma) is partially oxidized by NaIO₄ (0̸.33 g) in aqueous solution to form polyaldehyde dextran. The mixture is stirred in the dark for 1 hr at room temperature. The solution is concentrated by amicon cell (YM10̸ membrane MWCO=10̸,0̸0̸0̸) and purified by Sephadex G-25 column. The material is lyophilized to give 898 mg of white powder (89.8% yield).

The polyaldehyde dextran (80̸0̸ mg, 0̸.0̸2 mmole) is dissolved in 80̸ ml H₂O and then reacted with 1,3-diamino-2-hydroxypropane (20̸0̸ mg, 2.15 mmole) at room temperature for 24 hours. Sodium borohydride (11.8 mg, 0̸.311 mmole) is added and reacted at room temperature for 2 hours. The material is membrane filtered through YM-10̸ and XM-50̸ to eliminate the small molecules, and at the same time control the molecule weight at the selected ranges.

The level of the amino groups is assayed by TNBS (trinitrobenzene-sulfonic acid) using glucosamine as the reference material. The NH₂ level is found to be 10̸0̸/dextran.

### Example 2

### Preparation of Methotrexate/aminodextran intermediate

### (a) Activation of Methotrexate

In a dried Reacti-vial, 45.4 mg of methotrexate (0̸.1 mmole, Sigma) in 1 ml anhydrous DMF is introduced by syringe. A solution of N-hydroxysuccinimide (23 mg, 0̸.2 mmole, Sigma) in 7590̸ ul of anh. DMF and a solution of 1,3-dicyclohexylcarbodiimide (41.5 mg, 0̸.2 mmole, Sigma) in 750̸ ul of anh. DMF are followed. The reaction mixture is stirred in the dark at room temperature for 16 hours under anhydrous conditions. The white precipitate is centrifuged and the clear solution is stored in a sealed bottle at -20̸°C.

### (b) Reaction with Aminodextran

Aminodextran (10̸ mg, 2.5x10̸⁻⁴ mmole) is dissolved in 2 ml of PBS, pH 7.2. Activated MTX (125x10̸⁻⁴ mmole) is added gradually. The solution is stirred at room temperature for 5 hours and purified by Sephadex G-25 column. The void volume is collected and further dialyzed against reaction buffer. After lyophilization, 2.1 mg of product is obtained (21% yield). The methotrexate incorporation is determined by the absorption at 370̸ nm ( =650̸0̸) to be 38 Methotrexate/dextran.

### Example 3

### Preparation of antibody conjugate

### (a) Oxidation of Antibody

Anti-CEA monoclonal antibody is selectively oxidized by sodium metaperiodate to form aldehyde groups on the carbohydrate moiety. The procedure is as follows: Antibody (2 mg/ml) in PBS, pH 7.2 is reacted with 20̸ ul of sodium metaperiodate (2.84 ng/ml) in the dark at room temperature for 90̸ minutes. Ethylene glycol (2 ul) is then added. After 15 minutes, the oxidized antibody is purified by Sephadex G-25 column. The IgG fraction is collected and condensed to approximately 1 ml and used in the following conjugation.

### (b) Conjugation

The oxidized antibody (about 2 mg) is reacted with methotrexate/aminodextran intermediate prepared according to Example 2 (2.5 mg, 62.5 x 10̸⁻⁶ mmole) in PBS, pH 7.2. The solution is reacted at 4°C for 48 hours. The resultant Schiff base is stabilized by sodium cyanoborohydride (10̸-fold excess over the antibody). After sizing chromatography on Sephacryl S-30̸0̸, the conjugate appears as a symmetrical peak and is collected. The protein concentration is determined by Lowry assay to be 1.0̸5 mg (52.5% yield). The concentration of methotrexate is determined by the absorption at 370̸ nm ( =650̸0̸). The conjugate is found to contain 91 molecules of methotrexate per IgG molecule, which indicates that at least two dextran bridges are attached to the antibody.

The immunoreactivity of the conjugate is studied by flow-cytometry using indirect flourescence label technique. The data are compared with unmodified antibody and show that the conjugation by this method does not alter the immunoreactivity of the antibody.

### Example 4

### Preparation of Chelator Conjugate

An aminodextran of molecular weight 10̸0̸,0̸0̸0̸ is reacted with cyclic-DTPA to produce loaded carrier having an average of 10̸0̸ DTPA groups thereon. Condensation of the resultant loaded carrier with oxidized antibody, followed by reductive stabilization, gives a conjugate with 1-3 carrier groups per antibody, with negligible reduction in immunoreactivity. Loading of the conjugate with gadolinium(III) ions or with Indium-111, Gallium-67 or Technetium-99m produces highly loaded conjugate for scintigraphic or magnetic resonance imaging. Loading with, e.g., Yttrium-90̸ produces a therapeutically useful targeting agent.

### Example 5

### Cytotoxicity of MTX Conjugate

LS174T (colon adenocarcinoma) cells are treated with trypsin/EDTA, washed with complete media (RPMI-1640̸, 10̸% FCS, 10̸0̸0̸ u/ml penicillin, 10̸0̸0̸ ug/ml streptomycin, 25 mM Hepes), and added to microtiter well strips at 4x10̸ cells/well in 10̸0̸ ul, 6 replicates for each treatment. After 4 hrs, 37°C 6% CO₂, antibody-methotrexate conjugates are added along with the appropriate controls (free MTX, free MTX + free antibody). The cells are incubated for an additional 24 hours 37°C, 6% CO₂, at which time 0̸.1 uCi of 75-Se-selenomethionine is added for 16-18 hrs. Plates are washed 4 times. Individual wells are separated and counted in a gamma counter. The conjugate at a dose of about 3 uM causes about 50̸% of cell mortality.

### Example 6

### Tumor Therapy

A female patient having a small-cell carcinoma diffusely metastasized in both lobes of the lung is treated by intravenous injection of a solution of 10̸0̸ mg of MTX-AD-anti-CEA antibody conjugate in PBS, at a concentration of 10̸ mg/ml. The treatment is repeated for five successive days. CAT scans prior to treatment and 30̸ days after the last administration of the conjugate demonstrate 60̸% reduction in tumor volume.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims (Claims for the following Contracting State(s): BE, CH, DE, FR, GB, IT, LI, LU, NL, SE)

1. A soluble antibody conjugate, comprising a plurality of drug, toxin, chelator, boron addend or detectable label molecules covalently bound to an aminodextran carrier having at least one remaining free amine group, to form a loaded carrier, the loaded carrier in turn being covalently bound through at least one amine group to the carbohydrate portion of an antibody by a reduced Schiff base linkage; wherein the conjugate is soluble in human serum.

2. An antibody conjugate as claimed in claim 1, wherein said antibody is a monoclonal antibody.

3. An antibody conjugate as claimed in claim 1 or claim 2, wherein said antibody is an anti-cancer antibody.

4. An antibody conjugate as claimed in claim 3, wherein said anti-cancer antibody specifically binds to an antigen produced by or associated with a lung, breast, colorectal, liver, pancreatic, urogenital, stomach, kidney, lymphoid or epidermoid cancer.

5. An antibody conjugate as claimed in any one of claims 1 to 2, wherein said antibody specifically binds to an antigen produced by or associated with a non-cancerous infectious or inflammatory lesion.

6. An antibody conjugate as claimed in any one of claims 1 to 2, wherein said antibody specifically binds to an antigen characteristic of a specific type of normal organ or tissue.

7. An antibody conjugate as claimed in any one of claims 1 to 6, wherein said aminodextran is a condensation product of dextran and 1,3-diamino-2-hyroxypropane.

8. An antibody conjugate as claimed in any one of claims 1 to 7, wherein said aminodextran has about 50-150 amino groups thereon.

9. An antibody conjugate as claimed in claim 8, wherein said conjugate has about 25-50 methotrexate molecules per aminodextran.

10. The antibody conjugate of claim 9, wherein said conjugate has 1-3 methotextrate-aminodextran moieties per antibody.

11. An antibody conjugate as claimed in any one of claims 1 to 4 or 7 to 10, wherein said aminodextran carrier is loaded with a plurality of molecules of a cytotoxic agent.

12. An antibody conjugate as claimed in claim 11, wherein said cytotoxic agent is an anti-cancer drug.

13. An antibody conjugate as claimed in claim 12, wherein said anti-cancer drug is methotrexate, 5-fluorouracil, cycloheximide, daunomycin, doxorubicin, chlorambucil, trenimon, phenylenediamine mustard, adriamycin, bleomycin, cytosine arabinoside or cyclophosphamide.

14. An antibody conjugate as claimed in claim 11, wherein said cytotoxic agent is a toxin.

15. An antibody conjugate as claimed in claim 14, wherein said toxin is ricin or the A-chain thereof or pokeweed antiviral protein.

16. An antibody conjugate as claimed in any one of claims 1, 2 or 5 to 8, wherein said aminodextran carrier is loaded with a plurality of molecules of an antibiotic.

17. An antibody conjugate as claimed in claim 16, wherein said antibiotic is an antiviral, antifungal or antimicrobial drug.

18. An antibody conjugate as claimed in claim 16 or claim 17, wherein said antibiotic is mitomycin, actinomycin or analogues thereof.

19. An antibody conjugate as claimed in any one of claims 1 to 8, wherein the aminodextran carrier is loaded with a plurality of molecules of a boron addend.

20. An antibody conjugate as claimed in claim 19, wherein said boron addend is a carborane derivative.

21. An antibody conjugate as claimed in any one of claims 1 to 8, wherein said aminodextran carrier is loaded with a plurality of molecules of a chelator.

22. An antibody conjugate as claimed in claim 21, wherein said chelator is a chelator for a radiometal.

23. An antibody conjugate as claimed in claim 21, wherein said chelator is a chelator for a magnetic resonance enhancing metal ion.

24. An antibody conjugate as claimed in claim 21, wherein said chelator is:
(a) a derivative of ethylenediaminetetraacetic acid or diethylenetriaminepentaacetic acid;
(b) deferoxamine; or
(c) a bisthiosemicarbazone of a 1,2- or 1,3-dicarbonyl compound.

25. An antibody conjugate as claimed in any one of claims 1 to 8, wherein said aminodextran carrier is loaded with a plurality of molecules of a detectable label.

26. An antibody conjugate as claimed in claim 25, wherein said label is an enzyme, a fluorescent compound or an electron transfer agent.

27. A method of preparing soluble antibody conjugate as claimed in any one of claims 1 to 26, comprising the steps of:
(a) reacting dextran with 1,3-diamino-2-hydroxypropane to give an aminodextran carrier to which are covalently bound a plurality of drug, toxin, chelator, boron addend or detectable label molecules, the aminodextran carrier having at least one remaining free amine group, with an antibody having an oxidised carbohydrate portion to give a conjugate in which the carrier is covalently bound to the oxidised carbohydrate portion via a Schiff base linkage;
(b) reductively stabilising the resultant Schiff base adduct;
wherein the conjugate is soluble in human serum.

28. A scintigraphic imaging agent composition, comprising:
(a) a radiolabelled antibody which specifically binds to an antigen produced by or associated with a cancer or other pathological lesion; and
(b) a sterile, pharmaceutically acceptable injection vehicle, characterised in that said radiolabelled antibody is an antibody conjugate as claimed in claim 22.

29. A magnetic resonance imaging agent composition, comprising:
(a) an antibody labelled with a magnetic resonance enhancing metal ion, wherein said antibody specifically binds to an antigen produced by or associated with a cancer or other pathological lesion; and
(b) a sterile, pharmaceutically acceptable injection vehicle, characterised in that said labelled antibody is an antibody conjugate as claimed in claim 23.

30. A therapeutic composition for treatment of humans comprising:
(a) the antibody conjugate as claimed in any of claims 1 to 18; and
(b) a sterile, pharmaceutically acceptable injection vehicle.

31. A diagnostic composition for immunoassay or immunohistology, comprising an antibody conjugate as claimed in claim 1, wherein said aminodextran carrier is loaded with a plurality of molecules of a detectable label.

## Claims (Claims for the following Contracting State(s): AT)

1. A method of preparing a soluble antibody conjugate, comprising a plurality of drug, toxin, chelator, boron addend or detectable label molecules covalently bound to an aminodextran carrier having at least one remaining free amine group, to form a loaded carrier, the loaded carrier in turn being covalently bound through at least one amine group to the carbohydrate portion of an antibody by a reduced Schiff base linkage, wherein the conjugate is soluble in human serum, the method comprising the steps of:
(a) reacting dextran with 1,3-diamino-2-hydroxypropane to give an aminodextran carrier to which are covalently bound a plurality of drug, toxin, chelator, boron addend or detectable label molecules, the aminodextran carrier having at least one remaining free amine group, with an antibody having an oxidised carbohydrate portion to give a conjugate in which the carrier is covalently bound to the oxidised carbohydrate portion via a Schiff base linkage; and
(b) reductively stabilising the resultant Schiff base adduct.

2. A method as claimed in claim 1, wherein said antibody is a monoclonal antibody.

3. A method as claimed in claim 1 or claim 2, wherein said antibody is an anti-cancer antibody.

4. An method as claimed in claim 3, wherein said anti-cancer antibody specifically binds to an antigen produced by or associated with a lung, breast, colorectal, liver, pancreatic, urogenital, stomach, kidney, lymphoid or epidermoid cancer.

5. A method as claimed in any one of claims 1 to 2, wherein said antibody specifically binds to an antigen produced by or associated with a non-cancerous infectious or inflammatory lesion.

6. A method as claimed in any one of claims 1 to 2, wherein said antibody specifically binds to an antigen characteristic of a specific type of normal organ or tissue.

7. A method as claimed in any one of claims 1 to 6, wherein said aminodextran is a condensation product of dextran and 1,3-diamino-2-hyroxypropane.

8. A method as claimed in any one of claims 1 to 7, wherein said aminodextran has about 50-150 amino groups thereon.

9. A method as claimed in claim 8, wherein said conjugate has about 25-50 methotrexate molecules per aminodextran.

10. A method as claimed in claim 9, wherein said conjugate has 1-3 methotextrate-aminodextran moieties per antibody.

11. A method as claimed in any one of claims 1 to 4 or 7 to 10, wherein said aminodextran carrier is loaded with a plurality of molecules of a cytotoxic agent.

12. A method as claimed in claim 11, wherein said cytotoxic agent is an anti-cancer drug.

13. A method as claimed in claim 12, wherein said anti-cancer drug is methotrexate, 5-fluorouracil, cycloheximide, daunomycin, doxorubicin, chlorambucil, trenimon, phenylenediamine mustard, adriamycin, bleomycin, cytosine arabinoside or cyclophosphamide.

14. A method as claimed in claim 11, wherein said cytotoxic agent is a toxin.

15. A method as claimed in claim 14, wherein said toxin is ricin or the A-chain thereof or pokeweed antiviral protein.

16. A method as claimed in any one of claims 1, 2 or 5 to 8, wherein said aminodextran carrier is loaded with a plurality of molecules of an antibiotic.

17. A method as claimed in claim 16, wherein said antibiotic is an antiviral, antifungal or antimicrobial drug.

18. A method as claimed in claim 16 or claim 17, wherein said antibiotic is mitomycin, actinomycin or analogues thereof.

19. An method as claimed in any one of claims 1 to 8, wherein the aminodextran carrier is loaded with a plurality of molecules of a boron addend.

20. A method as claimed in claim 19, wherein said boron addend is a carborane derivative.

21. A method as claimed in any one of claims 1 to 8, wherein said aminodextran carrier is loaded with a plurality of molecules of a chelator.

22. A method as claimed in claim 21, wherein said chelator is a chelator for a radiometal.

23. A method as claimed in claim 21, wherein said chelator is a chelator for a magnetic resonance enhancing metal ion.

24. A method as claimed in claim 21, wherein said chelator is:
(a) a derivative of ethylenediaminetetraacetic acid or diethylenetriaminepentaacetic acid;
(b) deferoxamine; or
(c) a bisthiosemicarbazone of a 1,2- or 1,3-dicarbonyl compound.

25. A method as claimed in any one of claims 1 to 8, wherein said aminodextran carrier is loaded with a plurality of molecules of a detectable label.

26. A method as claimed in claim 25, wherein said label is an enzyme, a fluorescent compound or an electron transfer agent.

27. A process for the preparation of a therapeutic composition for treatment of humans, the process comprising admixing:
(a) a soluble antibody conjugate, comprising a plurality of drug, toxin, chelator, boron addend or detectable label molecules covalently bound to an aminodextran carrier having at least one remaining free amine group, to form a loaded carrier, the loaded carrier in turn being covalently bound through at least one amine group to the carbohydrate portion of an antibody by a reduced Schiff base linkage, wherein the conjugate is soluble in human serum; and
(b) a sterile, pharmaceutically acceptable injection vehicle.

## Patentansprüche (Patentansprüche für folgende(n) Vertragsstaat(en): BE, CH, DE, FR, GB, IT, LI, LU, NL, SE)

1. Lösliches Antikörper-Konjugat, aufweisend eine Vielzahl Von Arzneimittel-, Toxin-, Chelator-, Borzusatz- oder detektierbaren Markierungsmolekülen, kovalent gebunden an einen Aminodextran-Träger mit wenigstens einer verbleibenden freien Amingruppe, um einen beladenen Träger zu bilden, wobei der beladene Träger wiederum über wenigstens eine Amingruppe an den Kohlenhydratanteil eines Antikörpers durch eine Bindung einer reduzierten Schiffschen Base kovalent gebunden ist; wobei das Konjugat in Humanserum löslich ist.

2. Antikörper-Konjugat nach Anspruch 1, wobei der Antikörper ein monoklonaler Antikörper ist.

3. Antikörper-Konjugat nach Anspruch 1 oder Anspruch 2, wobei der Antikörper ein Anti-Krebs-Antikörper ist.

4. Antikörper-Konjugat nach Anspruch 3, wobei der Anti-Krebs-Antikörper spezifisch an ein Antigen bindet, das durch Lungen-, Brust-, colorektalen, Leber-, Pankreas-, urogenitalen, Magen-, Nieren-, lymphoiden oder epidermoiden Krebs erzeugt wird oder damit verbunden ist.

5. Antikörper-Konjugat nach einem der Ansprüche 1 bis 2, wobei der Antikörper spezifisch an ein Antigen bindet, das durch eine nichtkrebsartige infektiöse oder entzündliche Schädigung erzeugt wird oder damit verbunden ist.

6. Antikörper-Konjugat nach einem der Ansprüche 1 bis 2, wobei der Antikörper spezifisch an ein Antigen bindet, das charakteristisch für einen spezifischen Typ eines normalen Organs oder Gewebes ist.

7. Antikörper-Konjugat nach einem der Ansprüche 1 bis 6, wobei das Aminodextran ein Kondensationsprodukt aus Dextran und 1,3-Diamino-2-hydroxypropan ist.

8. Antikörper-Konjugat nach einem der Ansprüche 1 bis 7, wobei das Aminodextran etwa 50 bis 150 Aminogruppen besitzt.

9. Antikörper-Konjugat nach Anspruch 8, wobei das Konjugat etwa 25 bis 50 Methotrexat-Moleküle pro Aminodextran besitzt.

10. Antikörper-Konjugat nach Anspruch 9, wobei das Konjugat 1 bis 3 Methotrextat-Arninodextran-Anteile pro Antikörper besitzt.

11. Antikörper-Konjugat nach einem der Ansprüche 1 bis 4 oder 7 bis 10, wobei der Aminodextran-Träger mit einer Vielzahl von Molekülen eines cytotoxischen Mittels beladen ist.

12. Antikörper-Konjugat nach Anspruch 11, wobei das cytotoxische Mittel ein Anti-Krebs-Mittel ist.

13. Antikörper-Konjugat nach Anspruch 12, wobei das Anti-Krebs-Mittel Methotrexat, 5-Fluoruracil, Cycloheximid, Daunomycin, Doxorubicin, Chlorambucil, Trenimon, Phenylendiamin-mustard(gas) (-senfgas), Adriamycin, Bleomycin, Cytosin-arabinosid oder Cyclophosphamid ist.

14. Antikörper-Konjugat nach Anspruch 11, wobei das cytotoxische Mittel ein Toxin ist.

15. Antikörper-Konjugat nach Anspruch 14, wobei das Toxin Ricin oder die A-Kette davon oder ein antivirales Kermesbeeren-Protein ist.

16. Antikörper-Konjugat nach einem der Ansprüche 1, 2 oder 5 bis 8, wobei der Aminodextran-Träger mit einer Vielzahl von Molekülen eines Antibiotikums beladen ist.

17. Antikörper-Konjugat nach Anspruch 16, wobei das Antibiotikum ein antivirales, fungizides oder antimikrobielles Arzneimittel ist.

18. Antikörper-Konjugat nach Anspruch 16 oder Anspruch 17, wobei das Antibiotikum Mitomycin, Actinomycin oder ein Analoges davon ist.

19. Antikörper-Konjugat nach einem der Ansprüche 1 bis 8, wobei der Aminodextran-Träger mit einer Vielzahl von Molekülen eines Borzusatzes beladen ist.

20. Antikörper-Konjugat nach Anspruch 19, wobei der Borzusatz ein Caboran-Derivat ist.

21. Antikörper-Konjugat nach einem der Ansprüche 1 bis 8, wobei der Aminodextran-Träger mit einer Vielzahl von Molekülen eines Chelators beladen ist.

22. Antikörper-Konjugat nach Anspruch 21, wobei der Chelator ein Chelator für ein Radiometall ist.

23. Antikörper-Konjugat nach Anspruch 21, wobei der Chelator ein Chelator für ein Magnetresonanz-verstärkendes Metallion ist.

24. Antikörper-Konjugat nach Anspruch 21, wobei der Chelator ist:
(a) ein Derivat von Ethylendiamintetraessigsäure oder Diethylentriaminpentaessigsäure;
(b) Deferoxamin; oder
(c) ein Bisthiosemicarbazon einer 1,2- oder 1,3-Dicarbonylverbindung.

25. Antikörper-Konjugat nach einem der Ansprüche 1 bis 8, wobei der Aminodextran-Träger mit einer Vielzahl von Molekülen einer detektierbaren Markierung beladen ist.

26. Antikörper-Konjugat nach Anspruch 25, wobei die Markierung ein Enzym, eine Fluoreszenz-Verbindung oder ein Elektronen-Transfer-Mittel ist.

27. Verfahren zur Herstellung eines löslichen Antikörper-Konjugats nach einem der Ansprüche 1 bis 26, wobei das Verfahren die folgenden Stufen aufweist:
(a) Umsetzung von Dextran mit 1,3-Diamino-2-hydroxypropan unter Erhalt eines Aminodextran-Trägers, an den eine Vielzahl von Arzneimittel-, Toxin-, Chelator-, Borzusatz- oder detektierbaren Markierungsmolekülen kovalent gebunden wird, wobei der Aminodextran-Träger wenigstens eine verbleibende freie Amingruppe besitzt, mit einem Antikörper mit einem oxidierten Kohlenhydratanteil unter Erhalt eines Konjugats, in dem der Träger an den oxidierten Kohlenhydratanteil über eine Bindung einer Schiffschen Base kovalent gebunden wird;
(b) reduktives Stabilisieren des resultierenden Schiffsche Base-Abdukts;
wobei das Konjugat in Humanserum löslich ist.

28. Szintigraphische Abbildungsmittel-Zusammensetzung, aufweisend:
(a) einen radiomarkierten Antikörper, der spezifisch an ein Antigen bindet, das durch einen Krebs oder eine andere pathologische Schädigung erzeugt worden ist oder damit verbunden ist; und
(b) ein steriles, pharmazeutisch verträgliches Injektionsvehikel,
dadurch gekennzeichnet, das der radiomarkierte Antikörper ein Antikörper-Konjugat nach Anspruch 22 ist.

29. Magnetresonanz-Abbildungsmittel-Zusammensetzung, aufweisend:
(a) einen mit einem Magnetresonanz-verstärkenden Metallion markierten Antikörper, wobei der Antikörper spezifisch an ein Antigen bindet, das durch einen Krebs oder eine andere pathologische Schädigung erzeugt worden ist oder damit verbunden ist; und
(b) ein steriles, pharmazeutisch verträgliches Injektionsvehikel,
dadurch gekennzeichnet, daß der markierte Antikörper ein Antikörper-Konjugat nach Anspruch 23 ist.

30. Therapeutische Zusammensetzung zur Behandlung von Menschen, aufweisend:
(a) das Antikörper-Konjugat nach einem der Ansprüche 1 bis 18; und
(b) ein steriles, pharmazeutisch verträgliches Injektionsvehikel.

31. Diagnostische Zusammensetzung für Immunoassay oder Immunohistologie, aufweisend ein Antikörper-Konjugat nach Anspruch 1, wobei der Aminodextran-Träger mit einer Vielzahl von Molekülen einer detektierbaren Markierung beladen ist.

## Patentansprüche (Patentansprüche für folgende(n) Vertragsstaat(en): AT)

1. Verfahren zur Herstellung eines löslichen Antikörper-Konjugats, aufweisend eine Vielzahl von Arzneimittel-, Toxin-, Chelator-, Borzusatz- oder detektierbaren Markierungsmolekülen, kovalent gebunden an einen Aminodextran-Träger mit wenigstens einer verbleibenden freien Amingruppe, um einen beladenen Träger zu bilden, wobei der beladene Träger wiederum über wenigstens eine Amingruppe an den Kohlenhydratanteil eines Antikörpers durch eine Bindung einer reduzierten Schiffschen Base kovalent gebunden ist, wobei das Konjugat in Humanserum löslich ist, wobei das Verfahren die folgenden Schritte aufweist:
(a) Umsetzen von Dextran mit 1,3-Diamino-2-hydroxypropan unter Erhalt eines Aminodextran-Trägers, an den eine Vielzahl von Arzneimittel-, Toxin-, Chelator-, Borzusatz- oder detektierbaren Markierungsmolekülen kovalent gebunden wird, wobei der Aminodextran-Träger wenigstens eine verbleibende freie Amingruppe besitzt, mit einem Antikörper mit einem oxidierten Kohlenhydratanteil unter Erhalt eines Konjugats, in dem der Träger an den oxidierten Kohlenhydratanteil über eine Bindung einer Schiffschen Base kovalent gebunden ist; und
(b) reduktives Stabilisieren des resultierenden Schiffsche Base-Abdukts.

2. Verfahren nach Anspruch 1, wobei der Antikörper ein monoklonaler Antikörper ist.

3. Verfahren nach Anspruch 1 oder Anspruch 2, wobei der Antikörper ein Anti-Krebs-Antikörper ist.

4. Verfahren nach Anspruch 3, wobei der Anti-Krebs-Antikörper spezifisch an ein Antigen bindet, das durch einen Lungen-, Brust-, colorektalen, Leber-, Pankreas-, urogenitalen-, Magen-, Nieren-, lymphoiden oder epidermoiden Krebs erzeugt worden ist oder damit verbunden ist.

5. Verfahren nach einem der Ansprüche 1 bis 2, wobei der Antikörper spezifisch an ein Antigen bindet, das durch eine nicht-krebsartige infektiöse oder entzündliche Schädigung erzeugt worden ist oder damit verbunden ist.

6. Verfahren nach einem der Ansprüche 1 bis 2, wobei der Antikörper spezifisch an ein Antigen bindet, das charakteristisch für einen spezifischen Typ eines normalen Organs oder Gewebes ist.

7. Verfahren nach einem der Ansprüche 1 bis 6, wobei das Aminodextran ein Kondensationsprodukt aus Dextran und 1,3-Diamino-2-hydroxypropan ist.

8. Verfahren nach einem der Ansprüche 1 bis 7, wobei das Aminodextran etwa 50 bis 150 Aminogruppen besitzt.

9. Verfahren nach Anspruch 8, wobei das Konjugat etwa 25 bis 50 Methotrexat-Moleküle pro Aminodextran besitzt.

10. Verfahren nach Anspruch 9, wobei das Konjugat 1 bis 3 Methotrexat-Aminodextran-Anteile pro Antikörper besitzt.

11. Verfahren nach einem der Ansprüche 1 bis 4 oder 7 bis 10, wobei der Aminodextran-Träger mit einer Vielzahl von Molekülen eines cytotoxischen Mittels beladen wird.

12. Verfahren nach Anspruch 11, wobei das cytotoxische Mittel ein Anti-Krebs-Mittel ist.

13. Verfahren nach Anspruch 12, wobei das Anti-Krebs-Mittel Methotrexat, 5-Fluoruracil, Cycloheximid, Daunomycin, Doxorubicin, Chlorambucil, Trenimon, Phenylendiamin-mustard(gas) (-senfgas), Adriamycin, Bleomycin, Cytosin-arabinosid oder Cylophosphamid ist.

14. Verfahren nach Anspruch 11, wobei das cytotoxische Mittel ein Toxin ist.

15. Verfahren nach Anspruch 14, wobei das Toxin Ricin oder die A-Kette davon oder ein antivirales Kermesbeeren-Protein ist.

16. Verfahren nach einem der Ansprüche 1, 2 oder 5 bis 8, wobei der Aminodextran-Träger mit einer Vielzahl von Molekülen eines Antibiotikums beladen wird.

17. Verfahren nach Anspruch 16, wobei das Antibiotikum ein antivirales, fungizides oder antimikrobielles Arzneimittel ist.

18. Verfahren nach Anspruch 16 oder 17, wobei das Antibiotikum Mitomycin, Actinomycin oder ein Analoges davon ist.

19. Verfahren nach einem der Ansprüche 1 bis 8, wobei der Aminodextran-Träger mit einer Vielzahl von Molekülen eines Borzusatzes beladen wird.

20. Verfahren nach Anspruch 19, wobei der Borzusatz ein Carboran-Derivat ist.

21. Verfahren nach einem der Ansprüche 1 bis 8, wobei der Aminodextran-Träger mit einer Vielzahl von Molekülen eines Chelators beladen wird.

22. Verfahren nach Anspruch 21, wobei der Chelator ein Chelator für ein Radiometall ist.

23. Verfahren nach Anspruch 21, wobei der Chelator ein Chelator für ein Magnetresonanz-verstärkendes Metallion ist.

24. Verfahren nach Anspruch 21, wobei der Chelator ist:
(a) ein Derivat von Ethylendiamintetraessigsäure oder Diethylentriaminpentaessigsäure;
(b) Deferoxamin; oder
(c) ein Bisthiosemicarbazon einer 1,2- oder 1,3-Dicarbonylverbindung.

25. Verfahren nach einem der Ansprüche 1 bis 8, wobei der Aminodextran-Träger mit einer Vielzahl von Molekülen einer detektierbaren Markierung beladen wird.

26. Verfahren nach Anspruch 25, wobei die Markierung ein Enzym, eine Fluoreszenz-Verbindung oder ein Elektronen-Transfer-Mittel ist.

27. Verfahren zur Herstellung einer therapeutischen Zusammensetzung zur Behandlung von Menschen, wobei das Verfahren das Vermischen folgender Komponenten umfaßt:
(a) ein lösliches Antikörper-Konjugat, aufweisend eine Vielzahl von Arzneimittel-, Toxin-, Chelator-, Borzusatz-, oder detektierbaren Markierungsmolekülen, kovalent gebunden an einen Aminodextran-Träger mit wenigstens einer verbleibenden freien Amingruppe, um einen beladenen Träger zu bilden, wobei der beladene Träger wiederum über wenigstens eine Amingruppe an den Kohlenhydratanteil eines Antikörpers durch eine Bindung einer reduzierten Schiffschen Base kovalent gebunden ist, wobei das Konjugat in Humanserum löslich ist; und
(b) ein steriles, pharmazeutisch verträgliches Injektionsvehikel.

## Revendications (Revendications pour l'(les) Etat(s) contractant(s) suivant(s): BE, CH, DE, FR, GB, IT, LI, LU, NL, SE)

1. Conjugué d'anticorps soluble comprenant une multiplicité de molécules de médicaments, de toxines, de chélatants, de composés du bore ou de marqueurs détectables liées de manière covalente à un support aminodextrane ayant au moins un groupe amine libre restant pour former un support chargé, le support chargé étant à son tour lié de manière covalente par au moins un groupe amine à la partie glucidique d'un anticorps par une liaison de type base de Schiff réduite, le conjugué étant soluble dans le sérum humain.

2. Conjugué d'anticorps selon la revendication 1, dans lequel ledit anticorps est un anticorps monoclonal.

3. Conjugué d'anticorps selon la revendication 1 ou la revendication 2, dans lequel ledit anticorps est un anticorps anti-cancer.

4. Conjugué d'anticorps selon la revendication 3, dans lequel ledit anticorps anti-cancer se lie spécifiquement à un antigène produit par ou associé à un cancer du poumon, du sein, du colorectal, du foie, du pancréas, urogénital, de l'estomac, du rein, lymphoïde ou épidermoïde.

5. Conjugué d'anticorps selon l'une quelconque des revendications 1 ou 2, dans lequel ledit anticorps se lie spécifiquement à un antigène produit par ou associé à une lésion infectieuse ou inflammatoire non cancéreuse.

6. Conjugué d'anticorps selon l'une quelconque des revendications 1 ou 2, dans lequel ledit anticorps se lie spécifiquement à un antigène caractéristique d'un type spécifique d'organe ou tissu normal.

7. Conjugué d'anticorps selon l'une quelconque des revendications 1 à 6, dans lequel ledit aminodextrane est un produit de condensation du dextrane et du 1,3-diamino-2-hydroxypropane.

8. Conjugué d'anticorps selon l'une quelconque des revendications 1 à 7, dans lequel ledit aminodextrane comporte environ 50 à 150 groupes amino.

9. Conjugué d'anticorps selon la revendication 8, dans lequel ledit conjugué a environ 25 à 50 molécules de méthotrexate par aminodextrane.

10. Conjugué d'anticorps selon la revendication 9, dans lequel ledit conjugué a 1 à 3 entités méthotrexate-aminodextrane par anticorps.

11. Conjugué d'anticorps selon l'une quelconque des revendications 1 à 4 ou 7 à 10, dans lequel ledit support aminodextrane est chargé avec une multiplicité de molécules d'un agent cytotoxique.

12. Conjugué d'anticorps selon la revendication 11, dans lequel ledit agent cytotoxique est un médicament anti-cancer.

13. Conjugué d'anticorps selon la revendication 12, dans lequel ledit médicament anti-cancer est le méthotrexate, le 5-fluorouracile, le cycloheximide, la daunomycine, la doxorubicine, le chlorambucile, la trénimone, la moutarde de phénylènediamine, l'adriamycine, la bléomycine, l'arabinoside de cytosine ou le cyclophosphamide.

14. Conjugué d'anticorps selon la revendication 11, dans lequel ledit agent cytotoxique est une toxine.

15. Conjugué d'anticorps selon la revendication 14, dans lequel ladite toxine est la ricine ou sa chaîne A ou la protéine antivirale pokeweed.

16. Conjugué d'anticorps selon l'une quelconque des revendications 1, 2 ou 5 à 8, dans lequel ledit support aminodextrane est chargé avec une multiplicité de molécules d'un antibiotique.

17. Conjugué d'anticorps selon la revendication 16, dans lequel ledit antibiotique est un médicament antiviral, antifongique ou antimicrobien.

18. Conjugué d'anticorps selon la revendication 16 ou la revendication 17, dans lequel ledit antibiotique est la mitomycine, l'actinomycine ou des analogues de celles-ci.

19. Conjugué d'anticorps selon l'une quelconque des revendications 1 à 8, dans lequel le support aminodextrane est chargé avec une multiplicité de molécules d'un composé du bore.

20. Conjugué d'anticorps selon la revendication 19, dans lequel le composé du bore est un dérivé carborane.

21. Conjugué d'anticorps selon l'une quelconque des revendications 1 à 8, dans lequel ledit support aminodextrane est chargé avec une multiplicité de molécules d'un chélatant.

22. Conjugué d'anticorps selon la revendication 21, dans lequel ledit chélatant est un chélatant pour un radiométal.

23. Conjugué d'anticorps selon la revendication 21, dans lequel ledit chélatant est un chélatant pour un ion métallique augmentant la résonance magnétique.

24. Conjugué d'anticorps selon la revendication 21, dans lequel ledit chélatant est :
(a) un dérivé de l'acide éthylènediaminetétraacétique ou de l'acide diéthylènetriaminepentaacétique;
(b) la déféroxamine; ou
(c) une bisthiosemicarbazone d'un composé 1,2- ou 1,3-dicarbonylé.

25. Conjugué d'anticorps selon l'une quelconque des revendications 1 à 8, dans lequel ledit support aminodextrane est chargé avec une multiplicité de molécules d'un marqueur détectable.

26. Conjugué d'anticorps selon la revendication 25, dans lequel ledit marqueur est une enzyme, un composé fluorescent ou un agent de transfert d'électrons.

27. Procédé de préparation d'un conjugué d'anticorps soluble selon l'une quelconque des revendications 1 à 26, comprenant les étapes consistant à :
(a) faire réagir du dextrane avec du 1,3-diamino-2-hydroxypropane pour donner un support aminodextrane auquel sont liées de manière covalente une multiplicité de molécules de médicaments, de toxines, de chélatants, de composés du bore ou de marqueurs détectables, le support aminodextrane ayant au moins un groupe amine libre restant, avec un anticorps ayant une partie glucidique oxydée pour donner un conjugué dans lequel le support est lié de manière covalente à la partie glucidique oxydée par une liaison de type base de Schiff;
(b) stabiliser par réduction le produit d'addition de type base de Schiff résultant;
dans lequel le conjugué est soluble dans le sérum humain.

28. Composition d'agent d'imagerie scintigraphique, comprenant :
(a) un anticorps radiomarqué qui se lie spécifiquement à un antigène produit par ou associé à un cancer ou une autre lésion pathologique ; et
(b) un véhicule pour injection stérile, pharmaceutiquement acceptable, caractérisée en ce que ledit anticorps radiomarqué est un conjugué d'anticorps selon la revendication 22.

29. Composition d'agent d'imagerie par résonance magnétique comprenant :
(a) un anticorps marqué avec un ion métallique augmentant la résonance magnétique, dans lequel ledit anticorps se lie spécifiquement à un antigène produit par ou associé à un cancer ou une autre lésion pathologique; et
(b) un véhicule pour injection stérile, pharmaceutiquement acceptable, caractérisée en ce que ledit anticorps marqué est un conjugué d'anticorps selon la revendication 23.

30. Composition thérapeutique pour le traitement des humains, comprenant :
(a) le conjugué d'anticorps selon l'une quelconque des revendications 1 à 18, et
(b) un véhicule pour injection stérile, pharmaceutiquement acceptable.

31. Composition diagnostique pour immunodosage ou immunohistologie, comprenant un conjugué d'anticorps selon la revendication 1, dans lequel ledit support aminodextrane est chargé avec une multiplicité de molécules d'un marqueur détectable.

## Revendications (Revendications pour l'(les) Etat(s) contractant(s) suivant(s): AT)

1. Procédé de préparation d'un conjugué d'anticorps soluble comprenant une multiplicité de molécules de médicaments, de toxines, de chélatants, de composés du bore ou de marqueurs détectables liées de manière covalente un à un support aminodextrane ayant au moins un groupe amine libre restant, pour former un support chargé, le support chargé étant à son tour lié de manière covalente par au moins un groupe amine à la partie glucidique d'un anticorps par une liaison de type base de Schiff réduite, dans lequel le conjugué est soluble dans le sérum humain, le procédé comprenant les étapes consistant à :
(a) faire réagir du dextrane avec du 1,3-diamino-2-hydroxypropane pour donner un support aminodextrane auquel sont liées de manière covalente une multiplicité de molécules de médicaments, de toxines, de chélatants, de composés du bore ou de marqueurs détectables, le support aminodextrane ayant au moins un groupe amine libre restant, avec un anticorps ayant une partie glucidique oxydée pour donner un conjugué dans lequel le support est lié de manière covalente à la partie glucidique oxydée par une liaison de type base de Schiff ; et
(b) stabiliser par réduction le produit d'addition de type base de Schiff résultant.

2. Procédé selon la revendication 1, dans lequel ledit anticorps est un anticorps monoclonal.

3. Procédé selon la revendication 1 ou la revendication 2, dans lequel ledit anticorps est un anticorps anti-cancer.

4. Procédé selon la revendication 3, dans lequel ledit anticorps anti-cancer se lie spécifiquement à un antigène produit par ou associé à un cancer du poumon, du sein, colorectal, du foie, du pancréas, urogénital, de l'estomac, du rein, lymphoïde ou épidermoïde.

5. Procédé selon l'une quelconque des revendications 1 à 2, dans lequel ledit anticorps se lie spécifiquement à un antigène produit par ou associé à une lésion infectieuse ou inflammatoire non cancéreuse.

6. Procédé selon l'une quelconque des revendications 1 à 2, dans lequel ledit anticorps se lie spécifiquement à un antigène caractéristique d'un type spécifique d'organe ou tissu normal.

7. Procédé selon l'une quelconque des revendications 1 à 6, dans lequel ledit aminodextrane est un produit de condensation du dextrane et du 1,3-diamino-2-hydroxypropane.

8. Procédé selon l'une quelconque des revendications 1 à 7, dans lequel ledit aminodextrane comporte environ 50 à 150 groupes amino.

9. Procédé selon la revendication 8, dans lequel ledit conjugué a environ 25 à 50 molécules de méthotrexate par aminodextrane.

10. Procédé selon la revendication 9, dans lequel ledit conjugué a 1 à 3 entités méthotrexate-aminodextrane par anticorps.

11. Procédé selon l'une quelconque des revendications 1 à 4 ou 7 à 10, dans lequel ledit support aminodextrane est chargé avec une multiplicité de molécules d'un agent cytotoxique.

12. Procédé selon la revendication 11, dans lequel ledit agent cytotoxique est un médicament anti-cancer.

13. Procédé selon la revendication 12, dans lequel ledit médicament anti-cancer est le méthotrexate, le 5-fluorouracile, le cycloheximide, la daunomycine, la doxorubicine, le chlorambucile, la trénimone, la moutarde de phénylènediamine, l'adriamycine, la bléomycine, l'arabinoside de cytosine ou le cyclophosphamide.

14. Procédé selon la revendication 11, dans lequel ledit agent cytotoxique est une toxine.

15. Procédé selon la revendication 14, dans lequel ladite toxine est la ricine ou sa chaîne A ou la protéine antivirale pokeweed.

16. Procédé selon l'une quelconque des revendications 1, 2 ou 5 à 8, dans lequel ledit support aminodextrane est chargé avec une multiplicité de molécules d'un antibiotique.

17. Procédé selon la revendication 16, dans lequel ledit antibiotique est un médicament antiviral, antifongique ou antimicrobien.

18. Procédé selon la revendication 16 ou la revendication 17, dans lequel ledit antibiotique est la mitomycine, l'actinomycine ou des analogues de celles-ci.

19. Procédé selon l'une quelconque des revendications 1 à 8, dans lequel le support aminodextrane est chargé avec une multiplicité de molécules d'un composé du bore.

20. Procédé selon la revendication 19, dans lequel ledit composé du bore est un dérivé carborane.

21. Procédé selon l'une quelconque des revendications 1 à 8, dans lequel ledit support aminodextrane est chargé avec une multiplicité de molécules d'un chélatant.

22. Procédé selon la revendication 21, dans lequel ledit chélatant est un chélatant pour un radiométal.

23. Procédé selon la revendication 21, dans lequel ledit chélatant est un chélatant pour un ion métallique augmentant la résonance magnétique.

24. Procédé selon la revendication 21, dans lequel ledit chélatant est :
(a) un dérivé de l'acide éthylènediaminetétraacétique ou de l'acide diéthylènetriaminepentaacétique;
(b) la déféroxamine; ou
(c) une bisthiosemicarbazone d'un composé 1,2- ou 1,3-dicarbonylé.

25. Procédé selon l'une quelconque des revendications 1 à 8, dans lequel ledit support aminodextrane est chargé avec une multiplicité de molécules d'un marqueur détectable.

26. Procédé selon la revendication 25, dans lequel ledit marqueur est une enzyme, un composé fluorescent ou un agent de transfert d'électrons.

27. Procédé de préparation d'une composition thérapeutique pour le traitement des humains, le procédé comprenant le mélange :
(a) d'un conjugué d'anticorps soluble comprenant une multiplicité de molécules de médicaments, de toxines, de chélatants, de composés du bore ou de marqueurs détectables liées de manière covalente à un support aminodextrane ayant au moins un groupe amine libre restant pour former un support chargé, le support chargé étant à son tour lié de manière covalente par au moins un groupe amine à la partie glucidique d'un anticorps par une liaison de type base de Schiff réduite, le conjugué étant soluble dans le sérum humain, et
(b) d'un véhicule pour injection stérile, pharmaceutiquement acceptable.
